(19) Europäisches Patentamt
European Patent Office
Office européen des brevets

(11) Veröffentlichungsnummer: **0 018 321 B1**

(12) # EUROPÄISCHE PATENTSCHRIFT

(45) Veröffentlichungstag der Patentschrift: **16.06.82**

(51) Int. Cl.³: **C 07 D 487/16** // (C07D487/16, 251/00, 251/00, 251/00)

(21) Anmeldenummer: **80810103.4**

(22) Anmeldetag: **27.03.80**

(54) **Verfahren zur Herstellung von Cyamelurtrichlorid.**

(30) Priorität: **02.04.79 US 26262**

(43) Veröffentlichungstag der Anmeldung: **29.10.80 Patentblatt 80/22**

(45) Bekanntmachung des Hinweises auf die Patenterteilung: **16.06.82 Patentblatt 82/24**

(84) Benannte Vertragsstaaten: **BE CH DE GB**

(56) Entgegenhaltungen:
**DE-A-1 770 665**
**DE-A-1 770 667**
**US-A-3 089 875**

**CHEMICAL ABSTRACTS, Band 58, 1963, Columbus, Ohio, USA**
**H. SCHROEDER et el.: «Some reactions of cyameluric chloride»**

(73) Patentinhaber: **CIBA-GEIGY AG, Patentabteilung Postfach, CH-4002 Basel (CH)**

(72) Erfinder: **Gremmelmaier, Claude, Dr., Eichbergweg 6, CH-4147 Aesch (CH)**
Erfinder: **Riethmann, Jean, Dianastrasse 1, CH-4310 Rheinfelden (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 018 321 B1

ACTORUM AG

Verfahren zur Herstellung von Cyamelurtrichlorid

Die vorliegende Erfindung betrifft ein Verfahren zur Herstellung von Cyamelurtrichlorid.

Als Cyamelurtrichlorid bezeichnet man das 2,5,8-Trichlor-tri-s-triazin der Formel

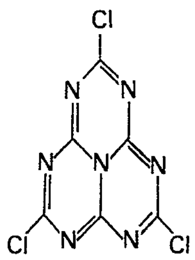

Für Cyamelurtrichlorid sind auch die Bezeichnungen Cyamelurchlorid oder Cyamelursäurechlorid gebräuchlich.

Cyamelurchlorid ist ein wertvolles Zwischenprodukt für die Herstellung verschiedenartiger Produkte, wie Gelierungsmitteln für aliphatische Kohlenwasserstoffe (vgl. US-Patent 3.089.875), fluorhaltigen Oxidantien, die als Zusatz zu Raketentreibstoffen verwendet werden können (vgl. US-Patent 3.202.659), Stabilisatoren für photographische Emulsionen (vgl. US-Patente 2.704.716 und 2.801.172) und Farbstoffen (vgl. DE-C-1.102.321).

Es ist bekannt, Cyamelursäurechlorid durch Umsetzung von Cyamelursäure oder einem Alkalicyamelurat mit Phosphorpentachlorid in einem geschlossenen System herzustellen (vgl. J. Amer. Chem. Soc. 62, 842 [1940]). Diese Umsetzung kann entweder im Bombenrohr bei 230° C (vgl. J. Org. Chem. 27, 4264 [1962]) oder in einem geeigneten, inerten Lösungsmittel, z.B. o-Dichlorbenzol (vgl. DE-C-1.102.321) durchgeführt werden. Die als Ausgangsmaterial benötigte Cyamelursäure bzw. das Alkalicyamelurat kann seinerseits durch alkalische Verseifung von Melon hergestellt werden, wobei zunächst das Alkalisalz der Cyamelursäure erhalten wird, aus dessen wässriger Lösung die Cyamelursäure durch Zugabe von Salzsäure abgeschieden werden kann (vgl. J. Amer. Chem. Soc. 61, 3420 [1939]) und Liebigs Ann. Chem. 73, 228 [1855]). Das zur Herstellung von Cyamelursäure benötigte Melon kann schliesslich durch Pyrolyse von Melamin bei 500° C (vgl. J. Appl. Chem. 9, 340 [1959]), durch langsames Erhitzen von Ammoniumthiocyanat (vgl. Z. Angew. Chem. 39, 1071 [1926]) oder durch Pyrolyse von Pseudothiocyanogen, das seinerseits durch Chlorierung von Natriumthiocyanat in wässriger Lösung erhalten wird (vgl. J. Amer. Chem. Soc. 61, 3420 [1939]), hergestellt werden.

Die Anwendung der vorgenannten Verfahren zur Herstellung von Cyamelurchlorid führt in jedem Fall zu einem vielstufigen Verfahren, dessen einzelne Stufen zum Teil schwierig durchzuführen und schwierig zu kontrollieren sind. Neben diesen technischen Schwierigkeiten besteht ein weiterer, mit der Anwendung der vorgenannten Verfahren verbundener Nachteil darin, dass infolge zahlreicher Nebenreaktionen Cyamelurchlorid nur in geringen Ausbeuten und in unreiner Form erhalten wird. Die vorgenannten Verfahren sind daher für eine technische Herstellung von Cyamelurchlorid ungeeignet.

Es ist daher das Ziel der vorliegenden Erfindung, ein Verfahren vorzuschlagen, das die Herstellung von reinem Cyamelurchlorid in einfacher Weise ermöglicht.

Es wurde gefunden, dass sich sehr reines Cyamelurchlorid bildet, wenn man Cyanurchlorid, Gemische von Cyanurchlorid und Chlorcyan oder Chlorcyan bei Temperaturen von 200 bis 700° C zusammen mit Sauerstoff über Aktivkohle leitet. Das erfindungsgemässe Verfahren zur Herstellung von Cyamelurchlorid ist daher dadurch gekennzeichnet, dass man Cyanurchlorid, Gemische von Cyanurchlorid und Chlorcyan oder Chlorcyan bei einer Temperatur von 200 bis 700° C zusammen mit Sauerstoff über Aktivkohle leitet und das entstandene Cyamelurchlorid durch Desublimation aus dem erhaltenen Gasgemisch abscheidet.

Als Aktivkohle kann vorteilhaft die üblicherweise zur Herstellung von Cyanurchlorid verwendbare Aktivkohle verwendet werden. Als Ausgangsmaterial für das erfindungsgemässe Verfahren dient in erster Linie Cyanurchlorid. Da jedoch Cyanurchlorid unter den angewendeten Reaktionsbedingungen an Aktivkohle teilweise zu Chlorcyan zurückgespalten wird, können auch Gemische aus Cyanurchlorid und Chlorcyan in die Reaktion eingesetzt werden. Da sich ferner unter den Reaktionsbedingungen aus Chlorcyan Cyanurchlorid bildet, kann auch reines Chlorcyan als Ausgangsmaterial dienen.

Innerhalb des angegebenen Temperaturbereichs von 200 bis 700° C sind Temperaturen von 300 bis 450° C bevorzugt. Die Kontaktzeiten und die Mengenverhältnisse der Reaktionspartner sind nicht kritisch und können in weiten Grenzen variiert werden. Es ist lediglich erforderlich, dass gleichzeitig mit dem Reaktionsgemisch eine geringe Menge Sauerstoff zugeführt wird. Der Sauerstoff kann entweder in reiner Form, in Form von Luft oder anderen sauerstoffhaltigen bzw. sauerstoffliefernden Gasen zugeführt werden. Vorzugsweise wird der Sauerstoff in Form von Luft zugeführt. Das gebildete Cyamelurchlorid kann wegen seines sehr niedrigen Dampfdrucks auf einfache Weise durch Desublimation in reiner Form aus dem Reaktionsgemisch abgetrennt werden.

Diese Desublimation wird vorteilhaft bei Temperaturen von 200 bis 250° C, vorzugsweise bei 200 bis 220° C vorgenommen. Das Cyanurchlorid und Chlorcyan enthaltende Restgas kann, gegebenenfalls nach Zugabe von neuem Sauerstoff in den Reaktor zurückgeführt werden. In manchen Fällen, insbesondere bei der Zufuhr von Sauerstoff in Form von Luft, ist es jedoch zweckmässig, das Restgas weiter aufzuarbeiten. Dies kann z.B. in der Weise geschehen, dass man zur quantitativen Abscheidung des Cyanurchlorids das

Restgas auf Raumtemperatur abkühlt. Aus dem nach Abscheidung von Cyanurchlorid erhaltenen Restgas kann dann das Chlorcyan entweder durch Kondensation oder durch Absorption mit einem geeigneten Lösungsmittel, z.B. Kohlenstofftetrachlorid, abgetrennt werden. Das so zurückgewonnene Cyanurchlorid und Chlorcyan kann dann zusammen mit frischem Sauerstoff erneut über den Aktivkohlekontakt geleitet werden.

Mit dem erfindungsgemässen Verfahren wird es möglich, ausgehend von leicht zugänglichen Rohstoffen in guter Ausbeute sehr reines Cyamelurchlorid herzustellen. Ein weiterer Vorteil des erfindungsgemässen Verfahrens besteht darin, dass das Cyamelurchlorid mit hoher Raumzeitausbeute hergestellt werden kann. Ein gewisser Nachteil des Verfahrens besteht lediglich darin, dass der Umsatz zu Cyamelurchlorid niedrig ist. Dies kann jedoch leicht dadurch ausgeglichen werden, dass man das nach Abtennung des Cyamelurchlorids erhaltene Restgas bzw. das in diesem Restgas enthaltene, nicht umgesetzte Cyanurchlorid und Chlorcyan in den Reaktor zurückführt.

Das erfindungsgemässe Verfahren wird durch die nachfolgenden Beispiele näher erläutert:

Beispiel 1

Als Reaktor dient ein senkrecht angeordnetes, elektrisch beheizbares Rohr aus Pyrexglas mit einer lichten Weite von 28mm, einer Länge von 500mm und einem konzentrischen Rohr von 5mm Durchmesser, in dem auf verschiedener Höhe Thermoelemente angebracht sind. Am unteren Ende des Reaktors befindet sich eine Vorlage, die zur Desublimation des Cyamelurchlorids dient und auf eine Temperatur von 200° C thermostatiert werden kann. Dieser ersten Vorlage ist eine zweite Vorlage nachgeschaltet, in der das Restgas zur Abscheidung von Cyanurchlorid auf Raumtemperatur abgekühlt werden kann. Die Vorlage zur Abscheidung von Cyanurchlorid ist eine mit Vorrichtungen zur Probeentnahme versehene Abgasleitung, die mit einer Absorptionskolonne verbunden ist, die mit wässriger Natronlauge beschickt ist.

Der Reaktor wird mit 20g (45ml) einer handelsüblichen, grobkörnigen Aktivkohle mit einer spezifischen Oberfläche (BET) von 1100 $m^2/g$ beschickt. Der Reaktor wird unter Spülung mit Luft (0,5 l/min) auf 400° C geheizt und während einer Stunde bei dieser Temperatur gehalten. Anschliessend werden kontinuierlich 1,16 kg/h Cyanurchlorid-Dampf und 30 l/h Luft bei 400° C über den Katalysator geleitet. Nach 370 Betriebsstunden haben sich in der ersten, auf 200° C thermostatierten Vorlage 1,108kg Cyamelurchlorid abgeschieden. Die Reinheit des Produkts beträgt gemäss Chlorbestimmung nach Wurzschmitt 99,5 bis 100% bzw. gemäss Stickstoffbestimmung nach Kjeldahl 98,6 bis 100%.

Die Raumzeitausbeute beträgt 66,5g Cyamelurchlorid pro Liter Katalysator und Stunde.

Der aus der Differenz von eingespeistem Cyanurchlorid und zurückgewonnenem Cyanurchlorid ermittelte, effektive Verbrauch an Cyanurchlorid beträgt 3,50 kg, was einer Ausbeute von 31,7 Gew.-% entspricht.

Die Analyse der nicht kondensierten Gase und der Waschlauge ergibt, dass diese Gase neben Phosgen, Chlor, Kohlendioxid und kleinen Mengen Kohlenmonoxid, Distickstoffmonoxid und Kohlenstofftetrachlorid noch 1,48 kg Chlorcyan enthalten. Daraus ergibt sich unter Berücksichtigung der Tatsache, dass für 1 Mol Cyamelurchlorid 7 Mol Chlorcyan oder ⅓ Mol Cyanurchlorid benötigt werden, eine Selektivität der Cyamelurchlorid-Bildung von 88%.

Beispiel 2

In dem in Beispiel 1 beschriebenen Reaktor werden Cyanurchlorid/Luft-Gemische verschiedener Zusammensetzungen bei verschiedenen Reaktionsbedingungen umgesetzt. Die Versuchsergebnisse sind in der folgenden Tabelle zusammengestellt:

| Versuch | Temperatur [° C] | Cyanurchlorid [kg/h] | Luft [l/h] | Molverhältnis Luft/Cyanurchlorid | Raumzeitausbeute [(Cyamelurchlorid) / l (Kat) · h] |
|---|---|---|---|---|---|
| 1 | 300 | 0,115 | 12 | 0,86 | 0,5 |
| 2 | 400 | 2,32 | 60 | 0,21 | 82,1 |
| 3 | 400 | 1,16 | 30 | 0,21 | 68,4 |
| 4 | 400 | 0,23 | 6 | 0,21 | 15,1 |
| 5 | 400 | 0,115 | 3 | 0,21 | 17,6 |
| 6 | 400 | 0,058 | 1,5 | 0,21 | 6,5 |
| 7 | 400 | 0,029 | 0,75 | 0,21 | 4,1 |
| 8 | 400 | 0,23 | 1,12 | 0,04 | 17,0 |
| 9 | 400 | 0,23 | 24 | 0,80 | 16,7 |
| 10 | 400 | 0,029 | 12 | 3,41 | 2,1 |
| 11 | 430 | 0,115 | 3 | 0,21 | 31,3 |
| 12 | 450 | 0,115 | 3 | 0,21 | 37,8 |
| 13 | 500 | 0,115 | 3 | 0,21 | 36,2 |
| 14 | 500 | 0,58 | 15 | 0,21 | 105,4 |
| 15 | 400 | 1,16 | 30* | – | 0 |

* Stickstoff anstelle von Luft

Die Versuchsergebnisse zeigen, dass die Luftmenge nur einen geringen Einfluss auf die Bildung von Cyamelurchlorid hat. Versuch 15 zeigt jedoch, dass zur Bildung von Cyamelurchlorid unbedingt Sauerstoff zugegen sein muss. Die Bildung von Cyamelurchlorid nimmt mit dem Cyanurchlorid-Durchsatz und der Temperatur zu. Bei Temperaturen über 430° C fällt jedoch die Selektivität der Reaktion langsam ab. Ausserdem wird bei diesen Temperaturen die Aktivkohle langsam zu Kohlendioxid oxidiert,

Beispiel 3

Der Reaktor gemäss Beispiel 1 wird mit 50 g Aktivkohle beschickt und, wie in Beispiel 1 beschrieben, auf 400° C aufgeheizt. Dann werden bei 400° C 0,06 kg/h Chlorcyan und 1,2 l/h Luft durch den Reaktor geleitet. Es werden 0,7 g/h Cyamelurchlorid gebildet.

**Patentansprüche**

1. Verfahren zur Herstellung von Cyamelurchlorid, dadurch gekennzeichnet, dass man Cyanurchlorid, Chlorcyan oder Gemische von Cyanurchlorid und Chlorcyan bei Temperaturen von 200 bis 700° C zusammen mit Sauerstoff über Aktivkohle leitet und das entstandene Cyamelurchlorid durch Desublimation aus dem erhaltenen Gasgemisch abscheidet.

2. Verfahren nach Anspruch 1, dadurch gekennzeichnet, dass man Cyanurchlorid bei einer Temperatur von 200 bis 700° C zusammen mit Sauerstoff über Aktivkohle leitet.

3. Verfahren nach Anspruch 1, dadurch gekennzeichnet, dass man bei einer Temperatur von 200 bis 450° C arbeitet.

4. Verfahren nach Anspruch 1, dadurch gekennzeichnet, das man die Desublimation des Cyamelurchlorids bei Temperaturen von 200 bis 250° C vornimmt.

5. Verfahren nach Anspruch 1, dadurch gekennzeichnet, dass man die Desublimation des Cyamelurchlorids bei Temperaturen von 200 bis 220° C vornimmt.

6. Verfahren nach Anspruch 1, dadurch gekennzeichnet, dass man das Cyanurchlorid und Chlorcyan enthaltende Restgas in den Reaktor zurückführt.

**Claims**

1. A process for the production of cyameluric chloride, which comprises passing cyanuric chloride, cyanogen chloride or mixtures of cyanuric chloride and cyanogen chloride, in the temperature range from 200° to 700° C, together with oxygen, over activated carbon, and separating the resultant cyameluric chloride by desublimation from the resultant mixture of gases.

2. A process according to claim 1, wherein cyanuric chloride is passed over activated carbon, together with oxygen, in the temperature range from 200° to 700° C.

3. A process according to claim 1, wherein the process is carried out in the temperature range from 200° to 450C.

4. A process according to claim 1, wherein the desublimation of the cyameluric chloride is effected in the temperature range from 200° to 250° C.

5. A process according to claim 1, wherein the desublimation of the cyameluric chloride is effected in the temperature range from 200° to 220° C.

6. A process according to claim 1, wherein the residual gas containing cyanuric chloride and cyanogen chloride is recycled to the reactor.

**Revendications**

1. Procédé de préparation de chlorure de cyaméluryle, caractérisé en ce qu'on fait passer du chlorure de cyanuryle, des mélanges de chlorure de cyanuryle et de chlorocyane ou du chlorocyane conjointement avec de l'oxygène sur du charbon actif à des températures de 200 à 700° C, puis on sépare le chlorure de cyaméluryle formé du mélange gazeux obtenu, par désublimation.

2. Procédé suivant la revendication 1, caractérisé en ce qu'on fait paser du chlorure de cyanuryle sur du charbon actif avec de l'oxygène à une température de 200 à 700° C.

3. Procédé suivant la revendication 1, caractérisé en ce qu'on travaille à une température de 200 à 450° C.

4. Procédé suivant la revendication 1, caractérisé en ce qu'on effectue la désublimation du chlorure de cyaméluryle à des températures de 200 à 250° C.

5. Procédé suivant la revendication 1, caractérisé en ce qu'on effectue la désublimation du chlorure de cyaméluryle à des températures de 200 à 220° C.

6. Procédé suivant la revendication 1, caractérisé en ce qu'on recycle le gaz résiduaire contenant du chlorure de cyanuryle et du chlorocyane dans le réacteur.